(19) Europäisches Patentamt / European Patent Office / Office européen des brevets

(11) **EP 2 642 417 B1**

(12) **EUROPEAN PATENT SPECIFICATION**

(45) Date of publication and mention
of the grant of the patent:
**08.05.2019 Bulletin 2019/19**

(51) Int Cl.:
***G06F 19/00*** *(2018.01)*

(21) Application number: **13159681.9**

(22) Date of filing: **18.03.2013**

(54) **Method for estimating energy loss of high polymer material**

Verfahren zum Einschätzen des Energieverlusts von Hochpolymermaterial

Procédé pour estimer la perte d'énergie de matériau polymère élevé

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.03.2012 JP 2012062358**

(43) Date of publication of application:
**25.09.2013 Bulletin 2013/39**

(73) Proprietor: **Sumitomo Rubber Industries, Ltd.
Kobe-shi,
Hyogo-ken (JP)**

(72) Inventor: **Ueno, Shinichi
Kobe-shi, Hyogo 651-0072 (JP)**

(74) Representative: **Manitz Finsterwald
Patent- und Rechtsanwaltspartnerschaft mbB
Martin-Greif-Strasse 1
80336 München (DE)**

(56) References cited:
**JP-A- 2007 107 968     JP-A- 2009 271 719**

• **Mossman M. A.: "Study of hysteretic damping in
small elastomeric structures", M.Sc. thesis, 2
October 1997 (1997-10-02), pages 1-160,
XP055341986, Canada Retrieved from the
Internet:
URL:https://open.library.ubc.ca/media/down
load/pdf/831/1.0088053/2 [retrieved on
2017-02-03]**

**Description**

**Background of the Invention**

The present invention relates to a computerized method for accurately estimating an energy loss of a high polymer material.

The energy loss of a high polymer material such as plastic and rubber is a very important physical quantity which affects various performances of products from the high polymer material. For example, a tire is a typical rubber product, and the energy loss of rubber used therein closely relates to grip performance, fuel consumption performance and the like of the tire. Therefore, it is important to exactly know and control the energy loss.

In general, in order to obtain, for example, a rubber compound having desired physical quantities, a development cycle - namely, designing of a rubber composition, trial production of the rubber compound, and measurement of the energy loss - is repeated until the desired physical quantities can be obtained.

In recent years, on the other hand, it has been proposed to estimate the energy loss of a high polymer material from results of a computerized simulation of the high polymer material, for example as disclosed in the following Patent documents 1 and 2.

[Patent document 1]    Japanese Patent Application Publication No. 2007-107968

[Patent document 2]    Japanese Patent Application Publication No. 2009-271719

JP 2009-271,719 A discloses a method for performing a simulation of a polymeric material using molecular dynamics calculations, includes: a model setting step S1 for setting a molecular chain model of a three-dimensional structure out of a polymeric material to be analyzed and based on its molecular structure, the molecular chain model including a finite number of particles representing atoms or its aggregate, and a coupling chain that couples the particles together and defines the relative position of each particle; a step S2 for simulating structural relaxation that stabilizes the molecular chain model based on the molecular dynamics calculations; a stress calculation step S3 for separating the molecular chain model after the structural relaxation into molecular chain portions, and calculating stresses occurring at each of the molecular chain portions; and a determination step S4 for determining principal portions where energy losses occur, based on the stresses at each of the molecular chain portions calculated in the stress calculation step.

In the methods disclosed in the Patent documents 1 and 2, roughly, the following steps (a)-(e) are implemented in this sequence: (a) a model of a molecular structure of the high polymer material is defined, (b) potentials are defined on the molecular structure model, (c) a structure relaxation calculation is made using a high polymer material model in which the molecular structure models are randomly disposed in a cell, (d) after the structure relaxation calculation has been done, by applying a cyclic deformation to the high polymer material model, the stress and strain are computed, and (e) a hysteresis loop is obtained from the computed results, and the energy loss is computed from the area of the hysteresis loop.

In such methods, however, there are problems as follows: in the step (d), sometimes, the calculation becomes unstable and abends during the calculation; and in the step (e), sometimes, the calculated value of the energy loss differs greatly from the actual measurement value.

The present inventor made a study and found that the calculation becomes unstable and abends when the oscillation cycle of the molecular structure model determined by the characteristic frequency of the potential, becomes substantially equal to the cycle of the cyclic deformation of the high polymer material model.

Summary of the Invention

It is therefor, an object of the present invention to provide a computerized method for estimating an energy loss of a high polymer material in which a deformation calculation is made only when the oscillation cycle of the molecular structure model does not coincide with the cycle of the cyclic deformation of the high polymer material model, and thereby the energy loss of the high polymer material can be accurately stably estimated.

According to the present invention, a computerized method for estimating an energy loss of a high polymer material, comprises:

a step in which a molecular structure model of a molecular structure of the high polymer material is defined, wherein the molecular structure model includes particle models each being of an atom or atoms and joining chains specifying relative positions between the particle models,

a step in which potentials between the particle models of the molecular structure model are defined,

a step in which a high polymer material model in which the molecular structure models are disposed in its cell is defined, wherein the cell is a space corresponding to a microstructural part of the high polymer material, and, based on the potentials and predetermined conditions, a structure relaxation calculation is made on the high polymer material model,

a step in which, after the structure relaxation calculation has been done, by applying a cyclic deformation to the high polymer material model, a deformation calculation to obtain stress and strain is made,

a step in which, based on results of the deformation calculation about the stress and strain, an energy loss is computed, wherein

the deformation calculation is made only when the cycle Ts of the cyclic deformation is out of a range from 0.8 to 1.2 times the oscillation cycle Tp which is the reciprocal of the characteristic frequency of each of the potentials.

Preferably, the structure relaxation calculation is made under such a condition that a density lower than the actual density of the high polymer material is defined on the high polymer material model. Preferably, the structure relaxation calculation for at least 10 picoseconds is made under a constant pressure and a constant temperature. Preferably, the deformation calculation is made for only the molecular structure models whose radii of inertia are less that 0.5 times a size of a cell.

## Brief Description of the Drawings

Fig. 1 is the structural formula of polybutadiene.
Fig. 2 is a flow chart of a simulation method as an embodiment of the present invention.
Fig. 3 shows a molecular structure model used in this embodiment.
Fig. 4 is a perspective view of a molecular structure model whose degree of polymerization is three.
Fig. 5 is a schematic perspective view for explaining a high polymer material model.
Fig. 6 is a graph showing a relationship between the density of the molecular structure model and the computing time of the structure relaxation calculation.
Fig. 7 is a flow chart of a deformation calculation in this embodiment.
Fig. 8(a) is a diagram of a cell for explaining the deformation calculation.
Fig. 8(b) is a graph for explaining a variation of the strain.
Fig. 9 is a graph showing a relationship between the strain and stress obtained by the deformation calculation.
Figs.10(a) and 10(b) are diagrams for explaining relationship between the radius of inertia of the molecular structure model and the size of the cell.

## Description of the Preferred Embodiments

An embodiment of present invention will now be described in detail in conjunction with accompanying drawings.

According to the present invention, the method for estimating an energy loss of a high polymer material is implemented by a computer.

The high polymer material encompasses at least rubber, resin and elastomer.

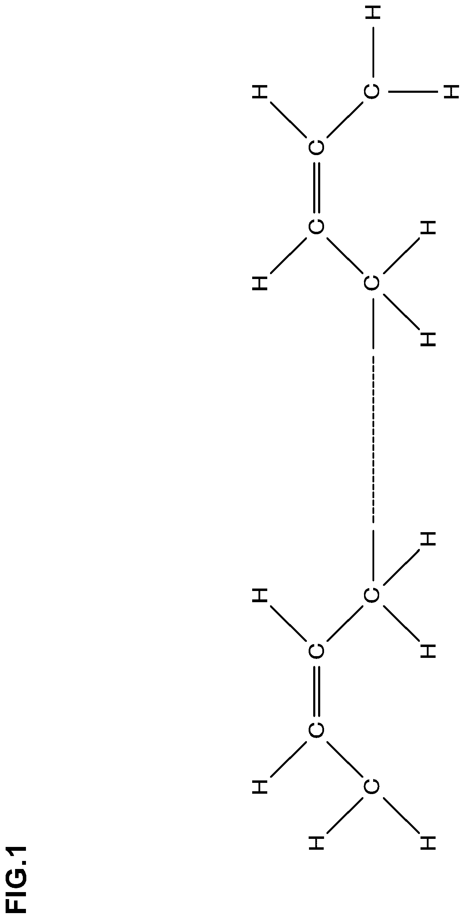

The high polymer material used in this embodiment is, as shown in Fig. 1, cis-1,4 polybutadiene (hereinafter, simply referred to as polybutadiene).

The polybutadiene is a polymer formed from the polymerization process of the monomer {-[CH2-CH=CH-CH2]-} made from a methylene group(-CH2-) and a methine group(-CH-).

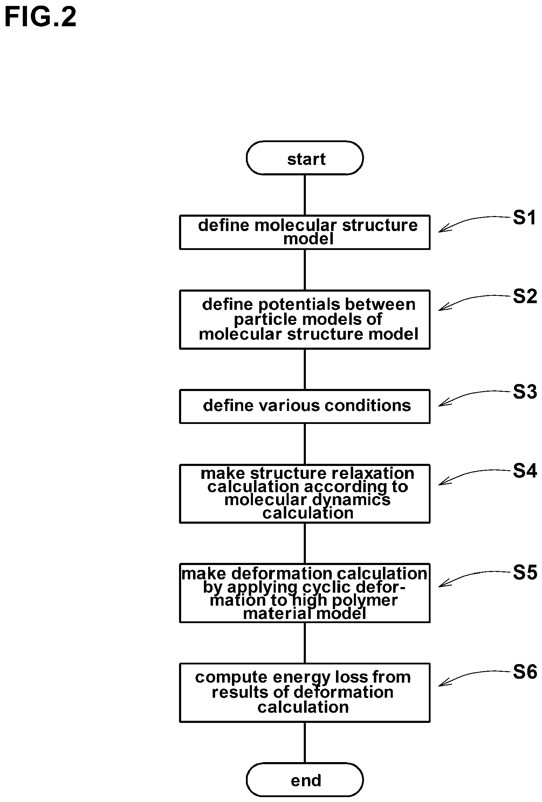

Fig. 2 shows a flow chart of the simulation method as an embodiment of the present invention.

In this embodiment, firstly, a molecular structure model of the high polymer material is generated by the computer and stored. (step S1).

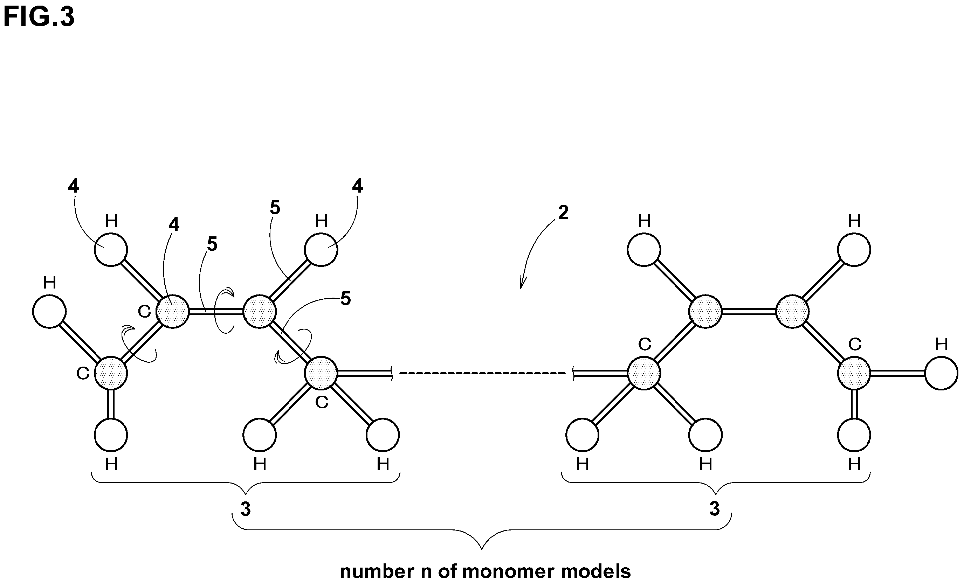

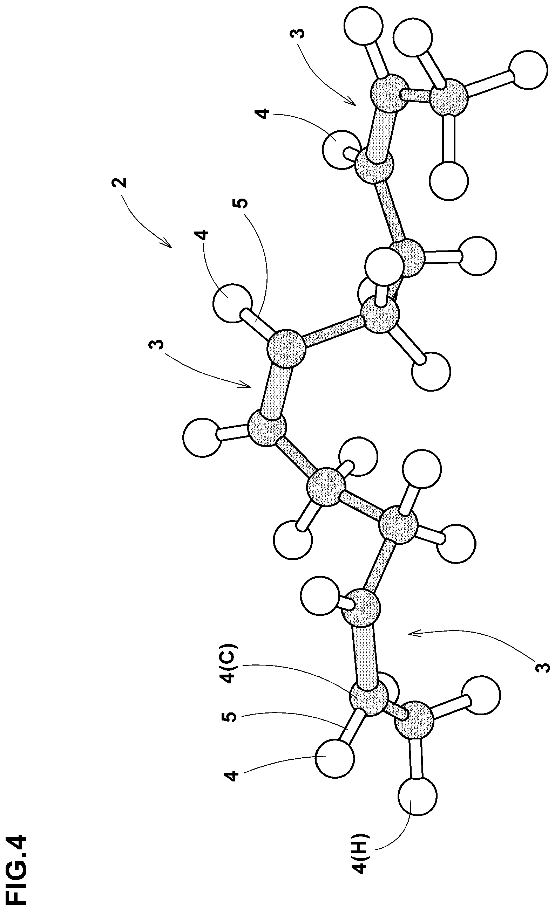

Fig. 3 and Fig. 4 show a molecular structure model 2 which represents a unit of a macromolecular chain of polybutadiene.

of course, the reality of the molecular structure model is numerical data capable of being dealt with in a molecular dynamics calculation by the computer.

The molecular structure model 2 is formed by linking a number (n) of monomer models 3 of a butadiene monomer. In the example shown in Fig. 4, the polymerization degree is three.

The monomer model 3 in this example is so called "full atom model" in which all of the atoms (C and H) are dealt with as particle models 4 in a computer simulation.

However, it is also possible that the monomer model 3 is so called "united atom model" in which a plurality of atoms are incorporated into one particle represented by one particle model.

In a computer simulation based on a molecular dynamics calculation, each particle model 4 incorporated in the molecular structure model 2 is dealt with as a material point in a motion equation. In other words, on each particle model 4, numerical data about its parameters - mass, volume, diameter, electric charge and/or initial coordinates - are defined and stored in the computer.

Between the particle models 4, joining chains 5 are defined to bind each other. The joining chains 5 are dealt with as springs, and for that purpose, for each of the joining chains 5, its equilibrium length and spring constant are defined and stored in the computer.

Each of the monomer models 3 is three-dimensional.

For each of the monomer models 3, bond lengths between particle models 4, bond angles each formed by three particle models 4 linked by joining chains 5, and dihedrals each formed by three adjacent particle models 4 of four particle models 4 linked by joining chains 5 are defined and stored in the computer.

when the monomer model 3 is subjected to an external force or an internal force, the bond lengths, bond angles and dihedrals may be changed, and accordingly, the three-dimensional structure of the molecular structure model 2 may be changed.

Such modeling is possible by the use of a software "J-OCTA" developed by JSOL corporation, for example.

Next, between the particle models 4, potentials are defined and stored in the computer. (step S2)

The potential is a function of an angle or a distance between the particles and used to calculate a force acting between two particle models 4.

Major potentials are as follows: Potential of bond stretching between particle models 4 linked by joining chain 5; Potential of bending formed by three continuous particle models 4; Potential of torsion formed by three or four continuous particle models 4; and Potential of Van der Waals' force between particle models 4 not linked with each other.

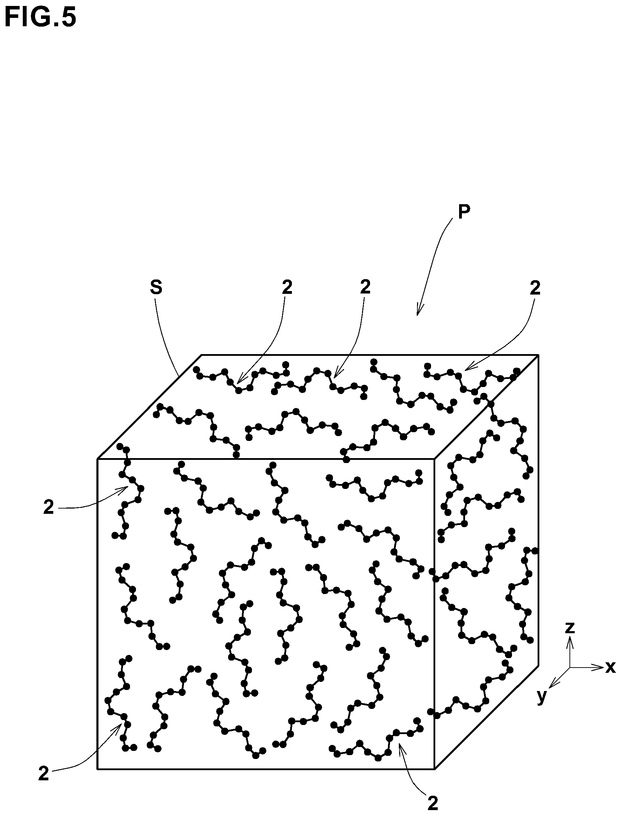

Next, the molecular structure models 2 initialized as above are arranged in a predetermined cell (space) S as shown in Fig. 5, and thereby a high polymer material model P is defined. Here, the molecular structure models 2 are randomly arranged to form a stable or metastable state.

Then, a structure relaxation calculation is carried out according to the potentials, predetermined conditions, and a molecular dynamics calculation. (step S3 and S4)

The cell S corresponds to a microstructural part of the high polymer material. In this example, the cell S is regular hexahedron. In the cell S, multiple molecular structure models 2 whose polymerization degree is three are randomly arranged.

Conditions for the structure relaxation calculation may be variously defined.

In this embodiment, the density of the high polymer material model P is set to a value less than the actual density of the polybutadiene. Here, the model's density is a quotient of the total mass of the molecular structure models 2 disposed in the cell S divided by the volume of the cell S. In this embodiment, the density of the molecular structure model is set to 0.01 g/cmA3 by default.

If a large number of molecular structure models 2 are disposed in the cell S from the start, there is a possibility that abnormal forces occur in the molecular dynamics calculation, and the calculation by the computer becomes unstable, when the molecular structure models 2 are overlapped each other. In this embodiment, however, since the structure relaxation calculation starts with the lower density, the probability of overlapping the molecular structure models 2 with each other is decreased and it is possible to stabilize the calculation.

It is desirable that the structure relaxation calculation for at least 10 picoseconds is made under a constant pressure and a constant temperature and under a periodic boundary condition.

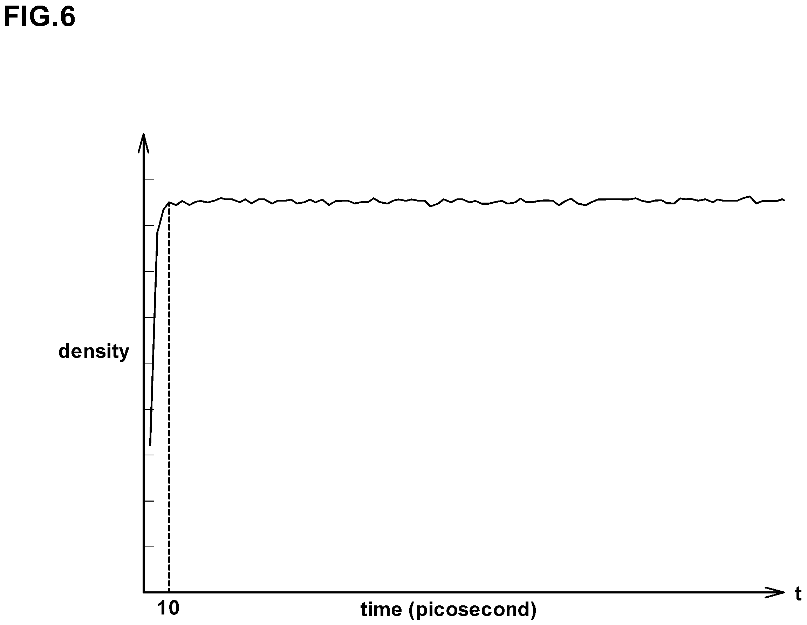

Fig. 6 shows a relationship between the density of the molecular structure model 2 and the calculation time as an example of the result of the structure relaxation calculation. As shown, by making the structure relaxation calculation for at least 10 picoseconds, the density of the high polymer material model is restored to its actual value.

As a result, a deformation calculation made on the high polymer material model P in the next step becomes stable.

Next, a deformation calculation is made by applying a cyclic deformation to the high polymer material model P stabilized through the structure relaxation calculation in order to calculate stress and strain, (step S5)

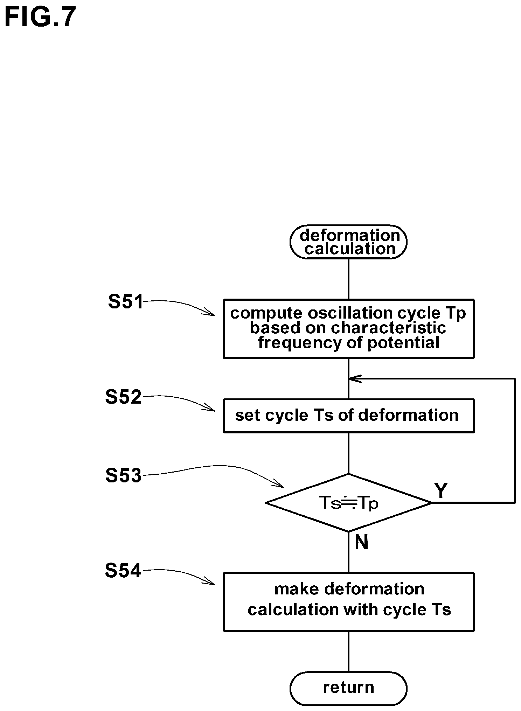

Fig. 7 shows a flow chart of an example of the deformation calculation step S5.

In this example, firstly, based on the characteristic frequency of each potential defined on the molecular structure models 2, an oscillation cycle Tp is calculated by the computer. (step S51)

In the case of the potential of bond stretching, for example, if the potential U of a C-C bond stretching is given by the following expression (1):

$$U = 0.5 * k * (x-x0)^2 \quad ---(1)$$

wherein

"k" is a spring constant,
"x" is a C-C bond length,
"x0" is the equilibrium length of the joining chain, then the characteristic frequency f of the bond stretching can be obtained by the following expression (2):

$$f=1/(2pi)*(k/M)^0.5 \quad ---(2)$$

wherein

"pi" is the circle ratio,
"M" is the mass of a carbon atom.

The oscillation cycle Tp is the reciprocal of a characteristic frequency.

Therefore, the oscillation cycle Tp of the characteristic frequency f of the potential of the bond stretching is obtained by the following expression (3):

$$Tp=1/f \quad ---(3)$$

Next, a cycle Ts of the deformation calculation made on high polymer material model P is set to a certain value. (step S52)

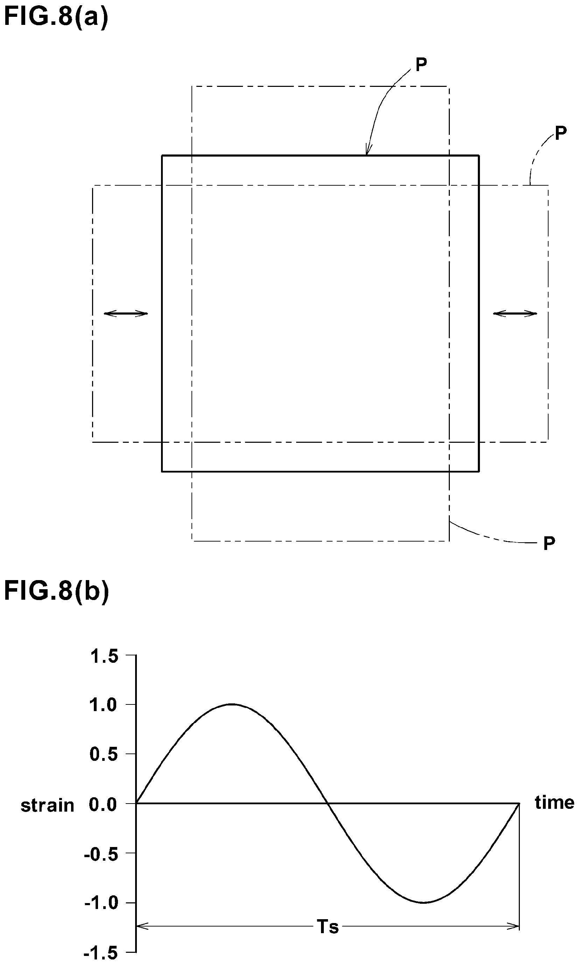

In the deformation calculation in this embodiment, as shown in Fig. 8(a), a tensile and compressive deformation is simulated, wherein the high polymer material model P is subjected to a uniaxial tensile strain and compressive strain of the same absolute value, and the course from the original state to original state (zero-strain to zero-strain) is regarded as one cycle.

Fig. 8(b) shows an example of the cyclic deformation of the high polymer material model P. In the figure, the strain is shown as a function of the time. In this example, the strain is varied like a sine wave. It is also possible to vary like a triangle wave.

Also, the magnitude of the strain, Poisson ratio and the like are set in addition to the cycle Ts.

Next, the computer judges if the cycle Ts is substantially equal to the oscillation cycle Tp. (step S53)

In the case that a plurality of different oscillation cycles Tp exist, the judgment is made with respect to each of the oscillation cycles Tp.
In this embodiment, if the ratio Ts/Tp is within a range of from 0.8 to 1.2, the cycle Ts is regarded as being substantially equal to the oscillation cycle Tp. It is of course possible to set the range differently.

If equal (Y in step S53), then the cycle Ts of the deformation calculation is set to a different value than the former value. (step S52)

If not equal (N in step S53), then the deformation calculation is made on the high polymer material model P with the cycle Ts. (step S54)

As explained above, if the oscillation cycle Tp becomes substantially equal to the cycle Ts, the calculation becomes unstable and abends. Further, the accuracy of the calculated results is greatly lowered. The reason is assumed to be abnormally large forces acting on the atoms due to large variations of the distances between the atoms occurring during the repetition of deformation. Such problem seems to be solved by making the deformation calculation for only one cycle (namely, not repeating the deformation calculation). But, when cyclic deformation is made using the molecular dynamics, regardless of whether or not the cycle Tp is equal to the characteristic frequency Ts, the influence of the deformation on the stress can not be recognized or it is hard to recognize for a short while after the beginning of the deformation. For this reason, to make the deformation calculation for only one cycle is not a realistic way.

In the present invention, therefore, before the deformation calculation is made on the high polymer material model P, the cycle Ts of the deformation of the high polymer material model P is compared with the oscillation cycle Tp determined based on the characteristic frequency of the potential of the molecular structure models 2, and if the cycle Ts is substantially not equal to the oscillation cycle Tp, the deformation calculation is made on the high polymer material model P. Therefore, in the deformation calculation, the cycle Ts is always different from the oscillation cycle Tp. Accordingly, the abend of the calculation and the deterioration of the calculation accuracy can be prevented.

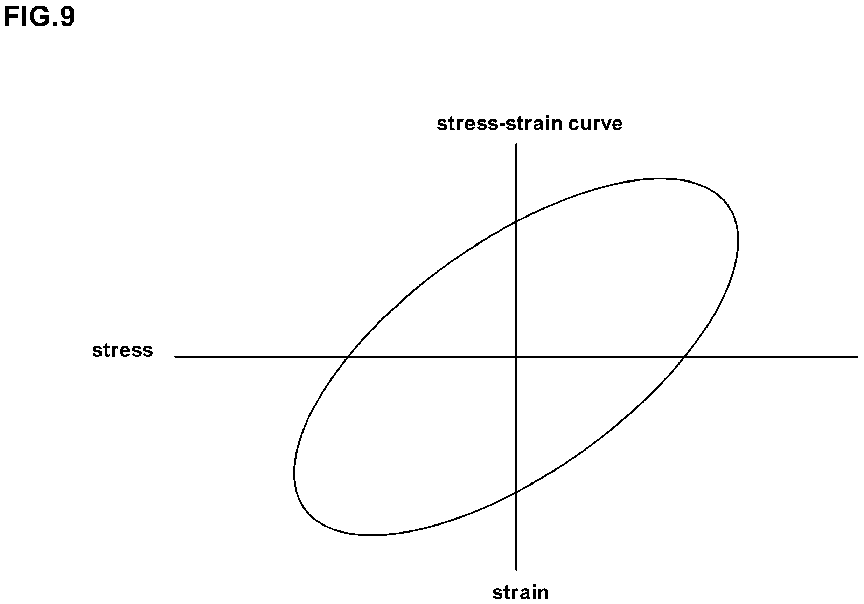

Fig. 9 shows a stress-strain curve (hysteresis loop) of the high polymer material model P obtained by the deformation calculation.

The energy loss of the high polymer material model P is obtained by computing the area surrounded by the hysteresis loop.

It is preferable that the deformation calculation is made, targeted at only the molecular structure models whose radii of inertia are less than 0.5 times the size of the cell S.

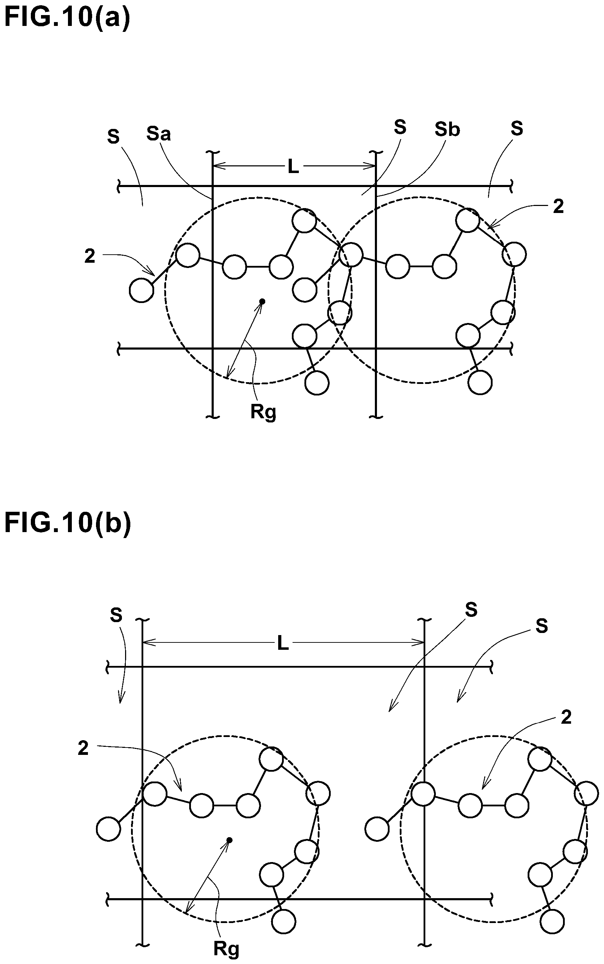

Fig. 10(a) shows an example where the radii of inertia Rg are more than 0.5 times the size L of the cell S (in this example, the length of a side of a cell S).

In the deformation calculation using the molecular structure models, a cycle boundary condition is defined where the cell S is repeated in a back-and-forth direction, a right-and-left direction and an up-and-down direction. In the cycle boundary condition, a boundary of a cell S abuts on a boundary of the next cell S.

If a molecular structure model 2 in a first cell S protrudes partially from a boundary Sa of the first cell S, then a part of a molecular structure model 2 in the next second cell S corresponding to the protruded part of the first cell protrudes from the opposite boundary Sb into the first cell S. Therefore, if the radius of inertia Rg is more than 0.5 times the size L of the cell S, there is a possibility that the particle models of the molecular structure models overlap each other, and as a resultan abnormal force is computed due to their potentials.

On the other hand, as shown in Fig. 10(b), if the radius of inertia Rg is less than 0.5 times the size L of the cell S, the above-mentioned interference or overlap can be avoided and an accurate calculation is possible.

**Comparison Tests**

In order to confirm the effects of the present invention, an energy loss was estimated according to the above described embodiment. The conditions were as follows:

the spring constant k of the potential of the bond stretching corresponding to the bond length = 918 [$\in/\sigma$^2],
the mass M of the particle model = 1.0 [m],
the characteristic frequency f = 4.82 [$1/\tau$], and
the oscillation cycle = 0.207 [$\tau$],
wherein

$\in$: unit of energy,
$\sigma$: unit of length,
m : unit of mass, and
$\tau$: unit of time.

In this embodiment, the cycle Ts of the deformation was set to 2.07 [$\tau$], namely 10 times the oscillation cycle Tp based on the characteristic frequency of the potential. As a result, a stress and strain curve like a hysteresis loop shown in Fig. 9 could be obtained.

For comparison, the cycle Ts was set to 0.207 [$\tau$], namely, a value equal to the oscillation cycle, and the calculation was made. In this case, the calculation was abnormally ended.

Further, it was also confirmed that when the ratios Ts/Tp were in a range of from 0.8 to 1.2, the calculations were abnormally ended with high probability.

As described above, in the method according to the present invention, the calculation can be made stably without being abnormally ended, and accurate estimations are possible.

**Claims**

1. A computerized method for estimating an energy loss of a high polymer material, comprising:

a step in which a molecular structure model of a molecular structure of the high polymer material is defined, wherein the molecular structure model includes particle models each being of an atom or atoms and joining chains specifying relative positions between the particle models,
a step in which potentials between the particle models of the molecular structure model are defined,
a step in which a high polymer material model in which the molecular structure models are disposed in its cell is defined, wherein the cell is a space corresponding to a microstructural part of the high polymer material, and, based on the potentials and predetermined conditions, a structure relaxation calculation is made on the high polymer material model,
a step in which, after the structure relaxation calculation has been done, by applying a cyclic deformation to the high polymer material model, a deformation calculation to obtain stress and strain is made,
a step in which, based on results of the deformation calculation about the stress and strain, an energy loss is obtained by computing the area surrounded by the hysteresis loop, wherein the deformation calculation is made only when the cycle Ts of the cyclic deformation is out of a range from 0.8 to 1.2 times the oscillation cycle Tp which is the reciprocal of the characteristic frequency of each of the potentials.

2. The method according to claim 1, wherein
the structure relaxation calculation is made under such a condition that a density lower than the actual density of the high polymer material is defined on the high polymer material model.

3. The method according to claim 1 or 2, wherein
the structure relaxation calculation for at least 10 picoseconds is made under a constant pressure and a constant temperature.

4. The method according to claim 1, 2 or 3, wherein
the deformation calculation is made for only the molecular structure models whose radii of inertia are less that 0.5 times a size of a cell.

**Patentansprüche**

1. Computergestütztes Verfahren zum Bestimmen eines Energieverlustes eines Hochpolymermaterials, umfassend:

einen Schritt, in dem ein Molekularstrukturmodell einer Molekularstruktur des Hochpolymermaterials definiert wird, wobei das Molekularstrukturmodell Partikelmodelle, die jeweils aus einem Atom oder Atomen bestehen, und Verbindungsketten umfasst, die relative Positionen zwischen den Partikelmodellen angeben,
einen Schritt, in dem Potentiale zwischen den Partikelmodellen des Molekularstrukturmodells definiert werden,
einen Schritt, in dem ein Hochpolymermaterialmodell, in welchem die Molekularstrukturmodelle in seiner Zelle angeordnet sind, definiert wird, wobei die Zelle ein Raum ist, der einem mikrostrukturellen Teil des Hochpolymermaterials entspricht, und, basierend auf den Potentialen und vorgegebenen Bedingungen, eine Strukturrelaxationsberechnung an dem Hochpolymermaterialmodell durchgeführt wird,
einen Schritt, in dem nach Durchführung der Strukturrelaxationsberechnung durch Anwenden einer zyklischen Verformung auf das Hochpolymermaterialmodell eine Verformungsberechnung zum Erhalten von Spannung und Dehnung durchgeführt wird,
einen Schritt, in dem, basierend auf den Ergebnissen der Verformungsberechnung über die Spannung und Dehnung, ein Energieverlust durch Berechnen der von der Hystereseschleife umgebenen Fläche erhalten wird, wobei
die Verformungsberechnung nur durchgeführt wird, wenn der Zyklus Ts der zyklischen Verformung außerhalb eines Bereichs vom 0,8- bis 1,2-fachen des Schwingungszyklus Tp liegt, der der Kehrwert der Eigenfrequenz eines jeden der Potentiale ist.

2. Verfahren nach Anspruch 1, wobei die Berechnung der Strukturrelaxation unter einer solchen Bedingung durchgeführt wird, dass eine Dichte, die niedriger als die tatsächliche Dichte des Hochpolymermaterials ist, an dem Hochpolymermaterialmodell definiert wird.

3. Verfahren nach Anspruch 1 oder 2, wobei die Berechnung der Strukturrelaxation für mindestens 10 Picosekunden unter einem konstanten Druck und einer konstanten Temperatur durchgeführt wird.

4. Verfahren nach Anspruch 1, 2 oder 3, wobei die Verformungsberechnung nur für die Molekularstrukturmodelle durchgeführt wird, deren Trägheitsradien weniger als das 0,5-fache der Größe einer Zelle betragen.

**Revendications**

1. Procédé mis en oeuvre en ordinateur pour estimer une perte d'énergie d'un matériau polymère élevé, comprenant :

une étape dans laquelle un modèle de structure moléculaire d'une structure moléculaire du matériau polymère élevé est défini, dans laquelle le modèle de structure moléculaire inclut des modèles de particules qui sont chacune un ou plusieurs atomes et des chaînes de jonction spécifiant des positions relatives entre les modèles de particules,
une étape dans laquelle des potentiels entre les modèles de particules du modèle de structure moléculaire sont définis,

une étape dans laquelle un modèle de matériau polymère élevé dans lequel les modèles de structure moléculaire sont disposées dans sa cellule est défini, ladite cellule étant un espace correspondant à une partie microstructurelle du matériau polymère élevé et, sur la base des potentiels et de conditions prédéterminées, un calcul de relaxation de structure est effectué sur le modèle de matériau polymère élevé,

une étape dans laquelle, après avoir effectué le calcul de relaxation de structure, en appliquant une déformation cyclique au modèle de matériau polymère élevé, on effectue un calcul de déformation pour obtenir les contraintes et les déformations,

une étape dans laquelle, sur la base du calcul de déformation concernant les contraintes et les déformations, une perte d'énergie est obtenue en calculant la superficie entourée par la boucle d'hystérésis, dans lequel le calcul de déformation est effectué uniquement quand le cycle Ts de la déformation cyclique est hors d'une plage de 0,8 à 1,2 fois le cycle d'oscillation Tp qui est la réciproque de la fréquence caractéristique de chacun des potentiels.

2. Procédé selon la revendication 1, dans lequel
le calcul de relaxation de structure est effectué dans une condition telle qu'une densité plus faible que la densité réelle du matériau polymère élevé est définie sur le modèle de matériau polymère élevé.

3. Procédé selon la revendication 1 ou 2, dans lequel
le calcul de relaxation de structure pour au moins 10 picosecondes est effectué sous une pression constante et une température constante.

4. Procédé selon la revendication 1, 2 ou 3, dans lequel
le calcul de déformation est effectué uniquement pour les modèles de structure moléculaire dont les rayons d'inertie sont inférieurs à 0,5 fois la taille d'une cellule.

FIG.1

# FIG.2

```
            ┌─────────────┐
            │    start     │
            └──────┬──────┘
                   │
    ┌──────────────────────────────┐        S1
    │   define molecular structure  │  ⟵
    │            model              │
    └──────────────┬───────────────┘
                   │
    ┌──────────────────────────────┐        S2
    │    define potentials between  │  ⟵
    │         particle models of    │
    │     molecular structure model │
    └──────────────┬───────────────┘
                   │
    ┌──────────────────────────────┐        S3
    │     define various conditions │  ⟵
    └──────────────┬───────────────┘
                   │
    ┌──────────────────────────────┐        S4
    │   make structure relaxation   │  ⟵
    │    calculation according to   │
    │      molecular dynamics       │
    │          calculation          │
    └──────────────┬───────────────┘
                   │
    ┌──────────────────────────────┐        S5
    │ make deformation calculation  │  ⟵
    │   by applying cyclic defor-    │
    │   mation to high polymer       │
    │        material model          │
    └──────────────┬───────────────┘
                   │
    ┌──────────────────────────────┐        S6
    │   compute energy loss from    │  ⟵
    │     results of deformation     │
    │          calculation           │
    └──────────────┬───────────────┘
                   │
            ┌─────────────┐
            │     end      │
            └─────────────┘
```

**FIG.3**

FIG.4

# FIG.5

# FIG.6

density

10     time (picosecond)     t

EP 2 642 417 B1

# FIG.7

deformation calculation

S51 — compute oscillation cycle Tp based on characteristic frequency of potential

S52 — set cycle Ts of deformation

S53 — $Ts \fallingdotseq Tp$ — Y

N

S54 — make deformation calculation with cycle Ts

return

# FIG.8(a)

# FIG.8(b)

FIG.9

stress-strain curve

stress

strain

## FIG.10(a)

## FIG.10(b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007107968 A

- JP 2009271719 A